Europäisches Patentamt

(19) European Patent Office

Office européen des brevets

(11) Veröffentlichungsnummer: **0 023 554**
B1

(12) # EUROPÄISCHE PATENTSCHRIFT

(45) Veröffentlichungstag der Patentschrift:
18.05.83

(51) Int. Cl.³: **H 05 B 3/36,** D 06 Q 1/04

(21) Anmeldenummer: **80103346.5**

(22) Anmeldetag: **16.06.80**

(54) **Verfahren zur Erhöhung der elektrischen Leistung von Heizelementen, die aus mit einem Metallüberzug versehenen textilen Flächengebilde bestehen, durch nachträgliche Oberflächenbehandlung.**

(30) Priorität: **04.07.79 DE 2926941**

(43) Veröffentlichungstag der Anmeldung:
**11.02.81 Patentblatt 81/6**

(45) Bekanntmachung des Hinweises auf die Patenterteilung:
**18.05.83 Patentblatt 83/20**

(84) Benannte Vertragsstaaten:
**AT BE CH DE FR GB IT LI NL SE**

(56) Entgegenhaltungen:
**DE-A-2 749 601**
**FR-A-2 340 016**
**GB-A-1 361 398**
**US-A-3 660 138**

(73) Patentinhaber: **BAYER AG, Zentralbereich Patente, Marken und Lizenzen, D-5090 Leverkusen 1, Bayerwerk (DE)**

(72) Erfinder: **Ebneth, Harold, Dr., Berta-von-Suttner-Strasse 61, D-5090 Leverkusen (DE)**
Erfinder: **Wolf, Gerhard Dieter, Dr., Wilhelm-Busch-Strasse 29, D-4047 Dormagen 5 (DE)**

Verfahren zur Erhöhung der elektrischen Leistung von Heizelementen, die aus einem mit einem Metallüberzug versehenen textilen Flächengebilde bestehen, durch nachträgliche Oberflächenbehandlung

In einer grossen Zahl technischer Anwendungsgebiete, z.B. in der Hydrokultur, Aquaristik, im Flugzeug- und im Automobilbau sucht man leichte, flexible, praktisch trägheitslos arbeitende, mit Kleinspannung ($\leqslant 50$ V) zu betreibende grossflächige Heizelemente, die gefahrlos zu handhaben sind und möglichst ihr textilelastisches Verhalten bewahren sollen.

Heizelemente in Form von Folien oder mit Kohle bzw. Graphit beschichteten Geweben sind z.B. aus R. Aigner u. P. Haasemann, „Kunststoffe 63" (1973), 11, 769-771, bekannt. Konventionell werden solche Heizelemente, z.B. mit 5- bis 7-litzigen Widerstandsdrähten, aufgebaut und mit Strom unterschiedlicher Spannung beheizt. Ferner sind aus der DE-A Nr. 2749601 heizbare Zeltmembranen bekannt, wobei auf das Zeltgewebe elektrische Leiter (Litze) gelegt werden und sowohl Gewebe als auch Leiter mit einem Tränkmittel durchtränkt bzw. benetzt werden, welches z.B. aus einer elektrisch leitenden Graphitlösung besteht. Diese Konstruktionen sind aber insofern nachteilig, als beim Brechen der Metalldrähte oder Knicken der Folien bzw. mit Kohlesintermaterial beschichteten Glasgeweben (etwa bei Dauerbiegebeanspruchung oder beim Transport) die Funktionstüchtigkeit gestört wird und eine Reparatur solcher geschädigter Heizdecken oder Heizmatten nicht mehr möglich ist.

Deshalb ist für spezielle Anwendungen ein textilelastisches, den Strom leitendes, textiles Flächengebilde, z.B. Gewebe, Gestrick, Gewirk, Vlies oder Papier, vorteilhaft gegenüber starren Gebilden oder Folien, da es z.B. geknickt, gefaltet, gerollt, gestaucht oder gereckt werden kann.

In der DE-OS Nr. 2743768 ist ein Verfahren beschrieben, wie ein leitendes, d.h. mit einem Metallüberzug versehenes textiles Flächengebilde hergestellt werden kann. Die Möglichkeit zur Anwendung dieser Gebilde als Flächenheizelemente ist in dieser DE-OS genannt.

Es wurde nun überraschend gefunden, dass man bei gleicher elektrischer Spannung die elektrische Leistung und somit auch die resultierende Oberflächentemperatur solcher Flächenheizelemente beträchtlich steigern kann, wenn man die Gebilde vor ihrem Einsatz, z.B. als Heizelemente, in an sich bekannter Weise mit einem Polymeren beschichtet, kaschiert, imprägniert, verklebt, verpresst oder kalandriert.

Die Erfindung betrifft daher ein Verfahren zur Erhöhung der elektrischen Leistung von Heizelementen, die aus einem mit einem Metallüberzug versehenen textilen Flächengebilde bestehen, das dadurch gekennzeichnet ist, dass das mit einem Metallüberzug versehene textile Flächengebilde einer Oberflächenbehandlung mit einem aus Polymer bestehenden Beschichtungsmaterial unterzogen wird.

Das textile, mit einem Metallüberzug vorzugsweise aus Nickel versehene Flächengebilde ist vorzugsweise ein Gestrick, Gewirk, Gewebe oder Vlies. Der Metallüberzug weist vorzugsweise eine Schichtdicke von mindestens 0,05 µm auf. Im allgemeinen sind Metallschichtdicken von 0,2 bis 0,8 µm besonders vorteilhaft. Die Metallisierung der textilen Flächengebilde erfolgt vorzugsweise nach dem in der DE-OS Nr. 2747768 beschriebenen Verfahren; sie kann aber auch nach anderen bekannten Metallisierungsverfahren erfolgen.

Die erfindungsgemässe Oberflächenbeschichtung bringt als weiteren Vorteil mit sich, dass die dünne Metallschicht vor mechanischer oder chemischer Schädigung geschützt ist.

Die elektrische Leitfähigkeit solcher Flächenheizelemente hängt bekanntlich von der Schichtdicke der Metallschicht auf den Fasern oder Fäden ab. Mit Zunahme der Schichtdicke tritt wegen Erhöhung des Querschnitts der Metallschicht eine Zunahme der elektrischen Leitfähigkeit ein.

Bereits bei einer Nickelschichtdicke von 0,05 µm ist die Leitfähigkeit so stark, dass sie für Heizzwecke im allgemeinen ausreicht. Mit dickeren Metallschichten, gemessen in Gramm Metall pro Flächeneinheit oder in Mikrometer Schichtdicke des abgeschiedenen Metalls, kann der Stromdurchfluss bei gleicher Spannung erhöht werden. Damit steigt die Heizleistung entsprechend an. Eine Nickelschicht von ca. 0,5 µm Dicke ist schon ausreichend, um z.B. einem Spinnfasergewebe (400×330 mm) aus aromatischen Polyamiden eine Oberflächentemperatur von 250° C bei 36 V/13,5 A zu ergeben. Dabei ist es gleichgültig, ob mit Gleich- oder Wechselstrom gearbeitet wird.

Die erfindungsgemässe Erhöhung der Heizleistung metallisierter Flächengebilde ergibt sich, wenn man z.B. eine Imprägnierung mit einem Polyesterurethanaddukt auf Basis von Toluylendiisocyanat mit Zusatz von Polyisocyanat vornimmt oder eine Kaschierung und Gelierung mit einem Weich-PVC auf der Kaschiermaschine oder eine Vakuumverformung mit Hilfe von Platten oder Folien aus thermoplastischen Kunststoffen (z.B. ABS-Polymerisaten, Polyolefinen, Polycarbonaten usw.) durchführt oder ganz einfach eine Beschichtung mit herkömmlichem, aus Polymer bestehendem Gummierungs- bzw. Beschichtungsmaterial vornimmt.

Beispiele für die praktische Anwendung sind mit Kleinspannung heizbare, z.B. mit Weich-PVC kaschierte Sitzbezüge oder Heizmatten z.B. für Aquarien, Hydrokulturen oder heizbare gummierte Textilien, z.B. für Taucher, Motorradnierengurte usw. Weitere Anwendungsmöglichkeiten sind z.B. Heizmatratzen für Krankenhäuser und Überzüge von Operationstischen.

Eine besondere Anwendungsmöglichkeit ist die des direkten Vakuumverfahrens auf Tiefziehmaschinen oder anderen Verformungsmaschinen, wie sie in der kunststoffverarbeitenden Industrie üblich sind. Das metallisierte textile Flächengebilde verbindet sich hierbei mechanisch-physikalisch

oder mit Hilfe eines Klebstoffes fest mit dem thermoplastischen Material und bleibt zwischen den beiden Platten bzw. Folien fest eingebaut. Gerade mit diesem Verfahren kann je nach Variation eine bis zu vierfache Steigerung des Stromdurchflusses in Ampere gemessen werden.

Auch ein Aufbringen von Schutzschichten mit Hilfe von Pressen, Kalandern, Walzen oder anderen gebräuchlichen Maschinen ist möglich und hat eine Verminderung des elektrischen Widerstandes verbunden mit einer Leistungssteigerung in Watt zur Folge.

*Beispiel 1:*

Ein mit 25 g/m² Nickel versehenes Gewebe aus handelsüblichen Polyacrylnitrilspinnfasern und ein mit 32 g/m² Nickel versehenes Gestrick aus porösen Polyacrylnitrilspinnfasern gemäss DE-OS Nr. 2554124 wurden mit einer Lösung eines Polyharnstoffpolyurethans in einem 7:3 Toluol/Isopropanol-Gemisch getränkt und anschliessend bei 40°C im Vakuum getrocknet. Durch unterschiedliche Konzentration der Lösung (2,5; 5; 10 und 20%) wurden verschieden dicke Überzüge auf den metallisierten Flächengebilden erreicht. Durch die Beschichtung mit diesem Polyharnstoffpolyurethan wurde der elektrische Widerstand der metallisierten textilen Flächengebilde deutlich verringert, in einigen Beispielen um mehr als die Hälfte des Ausgangswertes (s. Tab. 1).

Tabelle 1

| Konzentration der Beschichtung | 2,5% PU | | 5,0% PU | | 10% PU | | 20% PU | |
|---|---|---|---|---|---|---|---|---|
| | vorher[1] | nachher[1] | vorher[1] | nachher[1] | vorher[1] | nachher[1] | vorher[1] | nachher[1] |
| Polyacrylnitril-spinnfaser-gewebe | 5,9 | 4,7 | 10,9 | 6,0 | 4,8 | 3,2 | | |
| Gestrick aus porösen Poly-acrylnitril-spinnfasern | | | 11,6 | 6,6 | | | 7,6 | 3,4 |
| | | | 7,5 | 4,2 | | | 9,0 | 4,9 |

[1] Elektrischer Widerstand R vor und nach Beschichtung in Ohm ($\Omega$) bei einer elektrischen Spannung von 0,25 V (Messspannung)

Bei geringem Auftrag des Beschichtungsmittels bleibt der textile Charakter noch erhalten (z.B. aus 2,5%igen Lsg.). Mit zunehmender Beschichtungsdicke wird der Griff rauher und die Flächengebilde steifer. Die Abriebbeständigkeit des Nickels wird verbessert, bzw. es wird kein Abrieb mehr festgestellt. (Messungen mit dem Crockmeter Note 5 DIN 54021.)

*Beispiel 2:*

Das Polyacrylnitrilspinnfasergewebe und das poröse Polyacrylnitrilspinnfasergestrick aus Beispiel 1 wurden beidseitig mit einer 50 µm dicken, klaren PU-Folie bei 140°C verpresst. Diese Behandlung hatte eine Verringerung der elektrischen Widerstände auf sehr viel niedrigere Werte im Vergleich zu den unbehandelten Proben (s. Tab. 2) zur Folge. Mit dem Crockmeter wurde praktisch kein Nickelabrieb festgestellt.

Tabelle 2

| | Elektrischer Widerstand vor und nach dem Verpressen ($\Omega$) | | |
|---|---|---|---|
| | vorher | | nachher |
| Polyacrylnitrilspinnfasergewebe | 62,0 | (Schussrichtung) | 34,2 |
| | 22,5 | (Kettrichtung) | 6,3 |
| Poröses Polyacrylnitrilspinnfasergestrick | 9,4 | (längs zur Stäbchenrichtung) | 3,5 |
| | 37,5 | (quer zur Stäbchenrichtung) | 6,3 |

*Beispiel 3:*

Ein Baumwoll/Polyester-Fasergarngewebe in den Abmessungen 43×75 cm in Tuchbindung aus 50% Baumwolle und 50% Polyesterstapelfasern wurde ca. 0,7 µm stark vernickelt und die Oberflächentemperatur bei verschiedenen Spannungen gemessen: bei

12 V/1,3 A wurde eine Oberflächentemperatur von 25°C, bei

24 V/2,6 A wurde eine Oberflächentemperatur von 40°C, und bei

36 V/4,2 A wurde eine Oberflächentemperatur von 51°C gefunden.

Dann wurde die Probe mit einer nachträglich aufkaschierten Weich-PVC-Beschichtung versehen und die Oberflächentemperatur wurde erneut gemessen: bei

12 V/2 A wurde eine Oberflächentemperatur von 31°C, bei

24 V/3,7 A wurde eine Oberflächentemperatur von 61°C, und bei

36 V/6 A wurde eine Oberflächentemperatur von 74°C gefunden.

Es ist dabei gleichgültig, ob man Wechsel- oder Gleichstrom anlegt.

*Beispiel 4:*

Ein grobmaschiges vernickeltes Fasergarngewebe mit 45 g/m² Nickel aus handelsüblichen Polyacrylnitrilspinnfasern zeigte bei

24 V/0,6 A eine Oberflächentemperatur von 29°C.

Nach einer oberflächlichen, beidseitigen Kaschierung mit einer Weich-PVC-Kombination im Umkehrverfahren wurde bei

24 V/2,1 A eine Oberflächentemperatur von 45°C gemessen.

*Beispiel 5:*

Ein Polyacrylnitrilspinnfasergewebe in den Abmessungen 20×20 cm, einem Nickelgehalt von 9,43 Gew.%, einem elektrischen Widerstand R von 15,7 Ω in Kettrichtung und 32,4 Ω in Schussrichtung zeigte bei

12 V/0,05 A eine Oberflächentemperatur von 21°C, bei

24 V/0,1 A eine Oberflächentemperatur von 24°C, und bei

30 V/0,2 A eine Oberflächentemperatur von 28°C.

Das gleiche Muster wurde beidseitig mit einer 0,1 mm starken Polyäthylenfolie bei 100 kp/115°C 2 min lang verpresst. Danach wurden folgende Werte gemessen:

12 V/0,6 A ergaben eine Oberflächentemperatur von 37°C,

24 V/1,2 A ergaben eine Oberflächentemperatur von 48°C, und

30 V/1,5 A ergaben eine Oberflächentemperatur von 68°C.

Ein gleiches Muster wurde beidseitig mit einer 0,3 mm dicken Weich-PVC-Folie bei 100 kp/115°C 2 min lang verpresst. Bei

12 V/0,4 A wurde eine Oberflächentemperatur von 29°C, bei

24 V/0,8 A wurde eine Oberflächentemperatur von 38°C, und bei

30 V/1,2 A wurde eine Oberflächentemperatur von 58°C gemessen.

*Beispiel 6:*

Eine 3 mm dicke Platte eines ABS-Pfropfpolymerisats in den Abmessungen 400×300 mm wurde nach 2minütigem Vortrocknen auf einer Vakuumverformungsmaschine tiefgezogen. Die Platte wurde mit einem Infrarotstrahler (280°C-Strahlertemperatur) über den Erweichungspunkt aufgeheizt und dann mit Hilfe eines Vakuums 70 mm tief verformt. Die tiefgezogene Fläche betrug dann 310×210 mm.

In die verformte Platte wurde an den vertieften Stellen kleine Löcher gebohrt, damit bei einem zweiten Tiefziehvorgang der Unterdruck des Maschinenvakuums wirken kann. In die Vertiefung wurde ein vernickeltes Polyester/Baumwoll-Spinnfasergewebe im Mischungsverhältnis 50:50 eingelegt. Das Gewebe enthielt 29,5 Gew.% Nickel und einen Quadratwiderstand in Schussrichtung von 4,3 Ω, in Kettrichtung von 18,2 Ω. Die Abmessungen des Gewebeabschnittes waren 300×200 mm. Der obere Rand der Schale wurde mit einem Kleber versehen.

Anschliessend wurde eine zweite ABS-Pfropfpolymerisatplatte unter dem Infrarotstrahler über den Erweichungspunkt erwärmt und mit Hilfe des Maschinenvakuums in die vorbereitete Schale tiefgezogen. Dabei entstand ein guter Verbund zwischen dem vernickelten Gewebe und der aufgeheizten ABS-Folie einerseits und der mit dem Kleber bestrichenen Schalenoberkante der ersten, bereits verformten ABS-Schale.

Nach dem Tiefziehvorgang und Abkühlen wurde eine Verminderung des Quadratwiderstandes in Schussrichtung auf 2,6 Ω festgestellt.

Die mit Kleinspannung heizbare Kunststoffschale in Verbundbauweise erlaubte, bei 12 V/4,5 A eine Wassermenge von 3 l in kurzer Zeit auf 44°C zu erwärmen.

## Patentansprüche

1. Verfahren zur Erhöhung der elektrischen Leistung von Heizelementen, die aus einem mit einem Metallüberzug versehenen textilen Flächengebilde bestehen, dadurch gekennzeichnet, dass das mit einem Metallüberzug versehene textile Flächengebilde einer Oberflächenbehandlung mit einem aus Polymer bestehenden Beschichtungsmaterial unterzogen wird.

2. Verfahren nach dem Anspruch 1, dadurch gekennzeichnet, dass die Oberflächenbehandlung eine an sich bekannte Kaschierung, Imprägnierung, Verklebung, Verpressung, Kalandrierung oder Beschichtung mit einem Polymeren ist.

3. Verfahren nach den Ansprüchen 1 und 2, dadurch gekennzeichnet, dass das textile Flächengebilde ein Gestrick, Gewirk, Gewebe oder Vlies ist.

4. Verfahren nach den Ansprüchen 1 bis 3, dadurch gekennzeichnet, dass das textile Flächengebilde einen Metallüberzug mit einer Gesamtschichtdicke von mindestens 0,05 µm aufweist.

5. Verfahren nach den Ansprüchen 1 bis 4, dadurch gekennzeichnet, dass die zur Beheizung angelegte elektrische Spannung eine Gleich- oder Wechselspannung ist.

## Claims

1. Process for increasing the electrical power of heating elements which consist of a textile sheet provided with a metal coating, characterised in that the textile sheet provided with a metal coating is subjected to a surface treatment with a coating material consisting of a polymer.

2. Process according to claim 1, characterised in that the surface treatment is known lamination, impregnation, bonding, press moulding, calendering or coating with a polymer.

3. Process according to claims 1 and 2, characterised in that the textile sheet is a knitted, woven or non-woven fabric.

4. Process according to claims 1 to 3, characterised in that the textile sheet has a metal coating having a total thickness of at least 0.05 μm.

5. Process according to claims 1 to 4, characterised in that the electrical voltage applied for heating is a direct or alternating voltage.

## Revendications

1. Procédé pour élever la charge électrique d'éléments chauffants qui consistent en un objet textile plat muni d'un revêtement de métal, caractérisé en ce qu'on soumet l'objet textile plat muni du revêtement de métal à un traitement de surface à l'aide d'une matière d'enduction consistant en du polymère.

2. Procédé selon la revendication 1, caractérisé en ce que le traitement de surface est une opération, connue en soi, d'enduction, d'imprégnation, de collage, de compression, de calandrage ou de revêtement à l'aide d'un polymère.

3. Procédé selon les revendications 1 et 2, caractérisé en ce que l'objet textile plat est un tricot, une étoffe, un tissu ou un voile ou nappe.

4. Procédé selon les revendications 1 à 3, caractérisé en ce que l'objet textile plat présente un revêtement de métal ayant une épaisseur totale de couche d'au moins 0,05 μm.

5. Procédé selon les revendications 1 à 4, caractérisé en ce que la tension électrique utilisée pour le chauffage est une tension continue ou alternative.